(19) Europäisches Patentamt / European Patent Office / Office européen des brevets

(11) **EP 4 285 891 B1**

(12) **EUROPEAN PATENT SPECIFICATION**

(45) Date of publication and mention of the grant of the patent:
**23.04.2025 Bulletin 2025/17**

(21) Application number: **22717716.9**

(22) Date of filing: **27.01.2022**

(51) International Patent Classification (IPC):
*A61K 9/08* *(2006.01)*    *A61K 9/19* *(2006.01)*
*A61K 38/08* *(2019.01)*    *A61K 38/25* *(2006.01)*
*A61K 47/26* *(2006.01)*    *A61K 47/12* *(2006.01)*
*A61P 25/28* *(2006.01)*    *A61P 43/00* *(2006.01)*
*A61P 9/10* *(2006.01)*

(52) Cooperative Patent Classification (CPC):
**A61K 9/0019; A61K 9/08; A61K 9/19; A61K 38/08; A61K 47/12; A61K 47/26; A61P 9/10; A61P 25/28; A61P 43/00**

(86) International application number:
**PCT/CU2022/050001**

(87) International publication number:
**WO 2022/161554 (04.08.2022 Gazette 2022/31)**

(54) **PHARMACEUTICAL COMPOSITION CONTAINING GHRP-6**

PHARMAZEUTISCHE ZUSAMMENSETZUNG MIT GHRP-6

COMPOSITION PHARMACEUTIQUE COMPRENANT L'HORMONE DE CROISSANCE 6, GHRP-6

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **29.01.2021 CU 20210010**

(43) Date of publication of application:
**06.12.2023 Bulletin 2023/49**

(73) Proprietor: **Centro de Ingeniería Genética y Biotecnología**
**La Habana 11600 (CU)**

(72) Inventors:
• **SANTANA MILIÁN, Héctor, Jesús**
**La Habana 11600 (CU)**
• **HERNÁNDEZ BERNAL, Francisco**
**La Habana 11600 (CU)**
• **GONZALEZ BLANCO, Sonia**
**La Habana 11600 (CU)**
• **ZÁRATE RIVERA, Yasser**
**La Habana 10400 (CU)**
• **BACARDÍ FERNÁNDEZ, Dania, Mercedes**
**La Habana 17100 (CU)**

• **GARCIA DEL BARCO HERRERA, Diana**
**La Habana 11600 (CU)**
• **GONZALEZ GONZALEZ, Yaima**
**La Habana 11500 (CU)**
• **CASTRO ODIO, Fidel, Raul**
**La Habana 10400 (CU)**
• **BERLANGA ACOSTA, Jorge, Amador**
**La Habana 17100 (CU)**
• **GUILLÉN NIETO, Gerardo, Enrique**
**La Habana 11600 (CU)**
• **VALIENTE MUSTELIER, Juan**
**La Habana 10400 (CU)**

(74) Representative: **V.O.**
**P.O. Box 87930**
**2508 DH Den Haag (NL)**

(56) References cited:
• FERNÁNDEZ-MAYOLA M. ET AL.: "Growth hormone-releasing peptide 6 prevents cutaneous hypertrophic scarring: early mechanistic data from a proteome study", INTERNATIONAL WOUND JOURNAL, vol. 15, no. 4, 1 August 2018 (2018-08-01), UK, pages 538 - 546, XP055928488, ISSN: 1742-4801, Retrieved from the Internet <URL:https://www.ncbi.nlm.nih.gov/pmc/articles/PMC7949743/pdf/IWJ-15-538.pdf> DOI: 10.1111/iwj.12895

• HA I.S. ET AL.: "Degradation kinetics of growth hormone-releasing hexapeptide (GHRP-6) in aqueous solution", INTERNATIONAL JOURNAL OF PHARMACEUTICS, vol. 144, no. 1, 1996, pages 91 - 97, XP028783196, ISSN: 0378-5173, DOI: 10.1016/S0378-5173(96)04719-9

Remarks:
The complete document including Reference Table(s) and the Sequence Listing(s) can be downloaded from the EPO website

**Description**

**Technical Field**

The current invention is related to the pharmaceutical and biomedical industry, with the pharmaceutical formulation of peptides and proteins for improving its safety profile. In particular, the invention is related to pharmaceutical compositions of growth-hormone releasing peptide 6 (GHRP-6). It reveals pharmaceutical formulations of the peptide, suitable for its administration by parenteral route.

**Previous Art**

GHRP-6 is one of the most studied molecules in the family of secretagogues of growth hormone *(*Casanueva and Dieguez, Trends Endocrinol. Metab., 1999, 10: 30-38*)*. Its amino acid sequence is His-Trp-Ala-Trp-Phe-Lys-NH$_2$, where the second and fifth amino acids are D amino acids. It is a synthetic peptide, analogue of intestinal met-encephalin and gastric ghrelin, and it also competes with these agents for the somatrotroph adenohypophyseal receptors. These peptides act through the receptor coupled to protein G, known as receptor of the growth hormone secretagogues (GHS-R1a), whose natural ligand is ghrelin *(*Kojima et al., Nature, 1999, 402: 656-660*)*. It has been demonstrated there are additional binding sites for GHRP-6 in non-endocrine tissues, through its association to receptor CD36, which belongs to the family of scavenger or type B remover receptors *(*Bodart et al., Circ. Res., 1999, 85: 796-802*)*.

In studies conducted in animal models of ischemia/reperfusion, GHRP-6 has exhibited a potent cytoprotective effect *(*Murriel and Mochly, Arch. Biochem. Brophys., 2003, 420: 246-254). Preliminary pieces of evidence suggest the possibility that the mechanism by means of which tissue damages are lessened relates to the control of the cellular redox environment. Another form of cell death, like apoptosis, seems to be also prevented by GHRP-6 *(*Colonna et al., EUR. J. Pharmacol., 1997, 334: 201-207*;* Cibrián et al., Clin. Sci., 2006, 110: 563-573*)*. Its beneficial effect on the cardiovascular function has been evidenced in diseases like myocardial infarction and ischemic cardiopathology *(*Berlanga et al., Clin. Sci., 2007, 112: 241-250*;* Berlanga et al., Clin. Med. Insights Cardiol., 2017, 11: 1-9*)*. On the other hand, the combination of GHRP-6 with the epidermal growth factor (EGF) has turned out to be a promising therapy in the treatment of ischemic cerebral infarction *(*Subirós et al., Neurol. Res., 2016, 38 (3): 187-195*)*.

GHRP-6 has been administered in humans by parenteral route, mainly by intravenous route, with the purpose of inducing the release of the growth hormone. The dosage applied by intravenous route in humans oscillated between 1 and 2 μg/kg of weight *(*Leal-Cerro et al., EUR. J. Endocrinol., 1995, 132 (6): 712-715*);* Loche et al., J. Clin. Endocrinol. Metab., 1995, 80 (2): 674-678*)*. For new applications in humans, by parenteral route, pharmacological studies suggest that the therapeutic dosage of GHRP-6 is 100 times higher. The therapeutic dosage reported in a model of acute myocardial infarction in pigs was of 400 μg/kg *(*Berlanga et al., Clin. Sci., 2007, 112: 241-250*),* which represents a dosage between 200 and 400 times higher than the one used in humans, during the nineties. On the other hand, the therapeutic dosage of GHRP-6 reported in a model of ischemic cerebral infarction, in Mongolia gerbil (*Meriones unguiculatus*) was around 600 μg/kg *(*Subirós et al., Neurol. Res., 2016, 38 (3): 187-195*)*. In this last case, GHRP-6 was used in combination with EGF.

As to the safety in humans, during the administration of GHRP-6 by intravenous route, there are referred as safe the doses equal to or lower than 2 μg/kg. However, to attain an efficacious use of the peptide in the treatment of acute myocardial infarction and ischemic cerebral infarction, there are required much higher doses, in a range between 50 to 600 μg/kg. Likewise, for the said purpose, it is necessary to obtain a GHRP-6 formulation at a high concentration (from 1.0 to 10.0 mg/mL), which maintains the quality, stability and therapeutic properties, while minimizing the adverse and/or toxic effects. A high safety profile of the product is particularly necessary in the treatment of patients with acute myocardial infarction and stroke.

A particular problem of the formulations that include peptides or proteins at high concentrations is posed by its tendency towards aggregation. As result of aggregation, there may occur the precipitation of the product during storage, which may have an impact on the reproducibility of the dosage administered to the patients. Also, embolism may occur during the parenteral administration of particle-containing solutions.

Another general problem of pharmaceutical products is that adverse effects and/or toxicity might be found during the treatment of patients. These undesired events may be of different varieties, depending on characteristics of the active ingredient (metabolism, binding to proteins, etc.), formulation, route of administration and characteristics of the patients, among others *(*Ajayi et al., J. Clin. Pharmacol., 2000, 40 (10): 1093-1101*;* Choonara et al., Paediatr. Perinat. Drug Ther., 2001, 5: 12-18*)*. The preparation of concentrated formulations of peptides yielding an adequate therapeutic effect while maintaining at the minimum level the profile of adverse effects when applied by parenteral route, poses a challenge. It is necessary to develop a particular formulation for each peptide, since each one exhibits a unique *in-vivo* behavior.

Therefore, it is necessary to obtain of a stable pharmaceutical composition of GHRP-6 with the concentration of the peptide required, for its administration as therapeutic agent in several conditions, and at the same time exhibiting the safety profile demanded in humans.

**Description of the Invention**

The current invention solves the problem previously stated, by providing a pharmaceutical composition comprising GHRP-6 at a concentration between 1.0 mg/mL and 10.0 mg/mL, the pH-regulating buffer tartrate at 5.0 to 6.0, and trehalose. GHRP-6 counts with an amino acid sequence identified as SEQ ID NO: 1 in the Sequence Listing. This composition, which is revealed in the invention for the first time, exhibits a high safety profile, minimizing the adverse and toxic events detected for a preceding formulation, both in animals and humans.

Upon evaluating in rats a formulation of GHRP-6 that included citrate buffer at pH 6.0 and sodium chloride as isotonizing agent, elements of acute toxicity were found upon administering a dosage of the peptide that is 50 times the therapeutic dosage of 400 μg/kg. In said toxicological study, there were changes in the adrenal glands, consisting in focal areas of necrosis at the level of the fascicular zone with presence of tumescent cells and macro-vesicular steatosis. These signs are related to the administration of high doses of GHRP-6 contained in the citrate/NaCl formulation. Surprisingly, in toxicological studies conducted in rats, the formulation of the invention did not show signs of acute toxicity nor appearance of adverse effects in the animals as consequence of the application of doses 100 times higher than the therapeutic dosage, of 400 μg/kg of body weight. The formulation of the invention neither exhibited signs of toxicity at systemic level during the repeated administration of up to 50 times the therapeutic dosage (14 consecutive administrations). Furthermore, there was good local tolerance upon administering up to 10 times the therapeutic dosage, in a rabbit model broadly used for the toxicological evaluation of medicaments.

On the other hand, upon making a dosage scale up of the GHRP-6 formulation containing citrate buffer at pH 6.0 and sodium chloride, as isotonizing agent, in healthy voluntary individuals, occurred bradycardia in individuals who received the doses of 50 μg/kg and 100 μg/kg. Clinically, bradycardia may cause serious problems when the peptide is administered as medicament, particularly in patients suffering from pathologies like acute myocardial infarction or ischemic cerebral infarction. Surprisingly, the GHRP-6 formulation of the invention exhibited no signs of toxicity nor bradycardia was reported following the administration of doses of up to 100 μg/kg.

In the invention, the peptide GHRP-6 is at a concentration between 1.0 mg/mL and 10.0 mg/mL, in the pharmaceutical composition. In the nineties, the chemical stability of GHRP-6 was studied only at low concentrations (100 μg/mL approximately) with respect to pH/buffer system, ionic strength and temperature (Ha et al., Int. J. Pharm., 1996, 144: 91-97). In a contrasting manner, in the current invention, there was achieved stability of GHRP-6 at high concentrations, from 2.5mg/mL to 10mg/mL (25 to 100 times higher than the referred report), and in the presence of components in which the stability of the peptide had not been evaluated.

The pharmaceutical composition revealed in the invention, despite the high concentration of the peptide, exhibits high stability during storage under refrigerated conditions (5 ± 3°C). It also exhibits high stability at room temperature (25 ± 2°C), which allows removing the cold chain during the distribution of the medicament.

In an embodiment of the invention, the buffer has a concentration between 10 mmol/L and 100 mmol/L. The tartrate buffers that may be used are, basically, all those physiologically tolerated that are appropriate for reaching the desired pH values, such as sodium and potassium tartrate salts, or a mixture of them. The buffer consists, preferably, in one or more tartrate salts and/or acid free from the same (for instance, tartaric acid, sodium bitartrate, potassium bitartrate, anhydrous disodium tartrate, dihydrate disodium tartrate, disodium tartrate and tetrahydrate potassium, calcium tartrate). In a particular embodiment, tartrate buffer is made up by a sodium salt, a potassium salt or a mixture of both.

In an embodiment of the invention, the stabilizing compound, trehalose, is within the range from 2% to 10% (w/v). Preferably, the trehalose used is in D (+) form and in dihydrate form.

The formulation of the current invention may be presented as a liquid or in a dried form obtained by lyophilization. Therefore, the concentrations of the peptide, the buffer and the stabilizing agent refer to a concentration that is either in liquid form or that results from the suspension of a lyophilisate.

The lyophilization process occurs after sterilization (for instance, by means of filtration). It removes the solvent used in the system (water for injection, for example) down to levels lower than 5%, and even, reduces the same to values lower than 3% of residual humidity. The said process provides a GHRP-6 composition that may be stored at room temperature for long periods of time. The formulation is able to retain stability during storage, under the action of shear forces acting during the preparation, transportation and storage at high temperatures.

In a preferred embodiment of the invention, an aqueous trehalose solution in tartrate buffer is prepared; then, the dissolution of GHRP-6 in the previous solution takes place. The formulation may be optionally lyophilized. The lyophilized composition herein described is easily reconstituted once it becomes in contact with a sufficient amount of a pharmaceutically acceptable solvent. The lyophilized composition is reconstituted during less than two minutes. Suitable diluents include: water for injection; physiological saline solution; aqueous media in the presence of buffers; optionally, buffer solutions in the presence of sugars; vitamins; and synthetic polymers. In a preferred embodiment, the diluent is water for injection. The lyophilized composition may be reconstituted for producing a composition exhibiting the desired therapeutic concentration.

The medicament containing GHRP-6 along with other components is for use as cytoprotective agent in the treatment of tissues damaged due to the lack of blood irrigation. Therefore, in an embodiment of the invention, the medicament containing the indicated GHRP-6 formulation is for use as cytoprotective, cardio-protective, cardio-restorer or neuroprotective agent or as restorer of brain damage.

In an embodiment of the invention, the medicament is a kit of components that also comprises a second pharmaceutical composition. Therefore, it is also an object of the invention a kit of components that comprises the pharmaceutical composition comprising GHRP-6 at a concentration between 1.0 mg/mL and 10.0 mg/mL, tartrate buffer at pH 5.0 to 6.0 and trehalose.

In an embodiment of the invention, the pharmaceutical composition containing GHRP-6 is administered by intravenous, subcutaneous or intramuscular routes.

**Brief Description of the Figures**

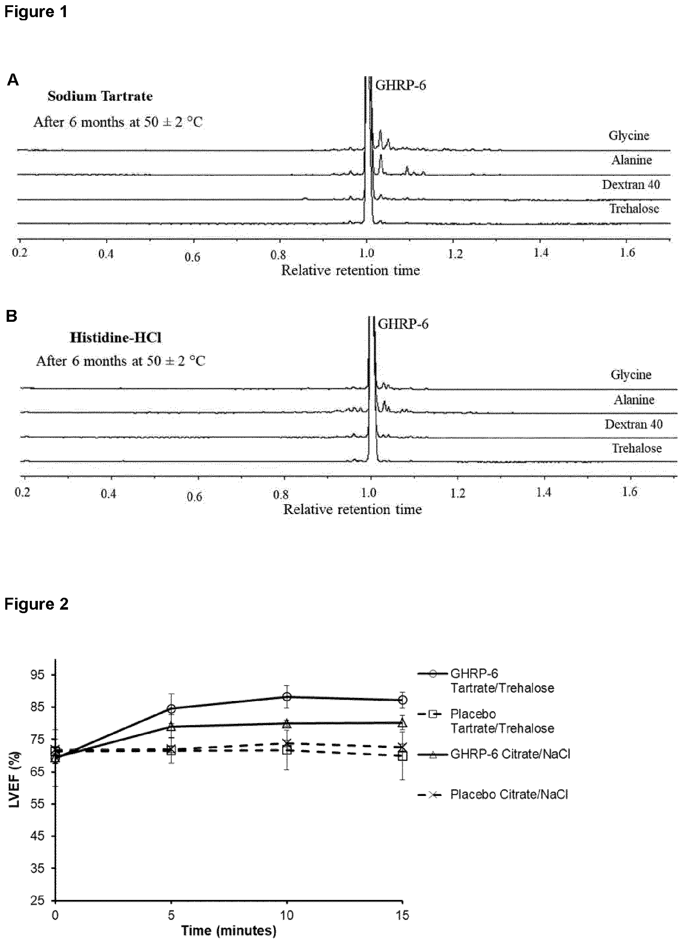

**Figure 1**. Reverse-phase high performance liquid chromatography (RP-HPLC) profile of GHRP-6 lyophilized in the presence of different excipients at 3% (w/v), in two buffers at 25 mmol/L and pH 5.5. (A) Sodium tartrate and (B) histidine-HCl. The concentration of the peptide was of 2.5 mg/mL. The samples were stored during six months, at $50 \pm 2°C$, prior to the chromatographic analysis.

**Figure 2.** Evaluation of the biological activity by means of the inotropic effect of two GHRP-6 formulations, in Balb/c mice ($n = 6$). As control of the experiment, the corresponding placebos of each formulation were used. The left ventricular ejection fraction (LVEF) was evaluated, by means of echocardiography. A dose of 100 $\mu$g of GHRP-6 per kilogram of body weight of the animal, or an equivalent volume of the corresponding placebo formulation, was administered by intraperitoneal route.

**Examples**

For all the studies conducted, GHRP-6 was used as active pharmaceutical ingredient (API), it has an amino acid sequence identified as SEQ IND No. 1 in the Sequence Listing. The peptide was obtained by means of the methodology of linear synthesis of peptides in solid phase, step by step (Atherton and Sheppard, 1989. Solid phase peptide synthesis: a practical approach, at: Coligan, J., Dunn, B., Ploegh H. (Eds.), Current Protocols in Protein Science. IRL Press, Oxford). The identity of the peptide batches was corroborated by means of mass spectrometry in electronebulization mode (ESI-MS), while the percentage of the fraction of the intact peptide was determined by means of RP-HPLC, resulting higher than the 98.0%.

**Example 1. Obtention of a Liquid Formulation of GHRP-6 in Citrate/Sodium Chloride.**

*Stability at Different pH Values, Ionic Species, Concentrations of the Buffer Solution and Concentration of Sodium Chloride*

The pH value of the pharmaceutical formulations is relevant for their administration by parenteral route. For studying the stability of GHRP-6 at different pH values, there was used the citrate-phosphate buffer (citric acid/$Na_2HPO_4$), at a concentration of 100mmol/L and pH values between 4.0 and 8.0. For the preparation of the different formulations, the API was dissolved until reaching the desired concentration of 2.5 mg/mL. The formulations were sterilized by filtration (through filters of 0.22 $\mu$m), and dispensed in 2R vials, at a ratio of 1.0 mL per vial. Representative samples of the different formulations were incubated at 60°C and the stability of the peptide was evaluated by means of RP-HPLC following storage at the said temperature during different time intervals.

The percentage of the fraction of the intact peptide was evaluated in RP-HPLC, in a C18 column (4.6 x 150mm, 5$\mu$m), (Vydac, United States of America). As mobile phase, there was used the mixture of a solution of water for injection with trifluoroacetic acid (TFA) at 0.1% (v/v), (Solution A), and acetonitrile with TFA at 0.05% (v/v), (Solution B). For separating the degradation products formed during the stress study, there was used a gradient from 0 to the 60% of B in A, during 45 minutes, at a work temperature of 34°C. GHRP-6 concentration was determined by means of the Lambert-Berr law, starting from the reading of absorbance at 280nm and mass extinction coefficient ($\varepsilon^{1\%}$ 280nm) of 146.8 mLmg$^{-1}$cm$^{-1}$, experimentally determined. During the storage of the GHRP-6 formulations (at 2.5 mg/mL) in sodium citrate-phosphate buffer at 60°C, the highest purity values in RP-HPLC were obtained at pH 6.0.

Next, there was studied the stability of the peptide (2.5 mg/mL) at the same pH value, in sodium acetate, sodium citrate, sodium phosphate and sodium citrate-phosphate buffers, at concentrations of the buffer of 25, 50 and 100 mmol/L. In the phosphate, citrate and citrate-phosphate buffers, there was observed a catalytic effect of the same in the degradation of the peptide; with the increase of concentration of the buffers, the peptide's purity decreased. With the use of the sodium acetate buffer, no catalytic effect was observed. According to the RP-HPLC analysis, the highest purity values of the peptide were observed with the use of the sodium citrate buffer at pH 6.0 and a concentration of 25 mmol/L.

Finally, with the purpose of evaluating sodium chloride as a isotonizing agent, there was evaluated the effect of its concentration (2.5, 5.0 and 10.0 mg/mL) on GHRP-6 stability. This study was conducted in sodium citrate buffer at pH 6.0 (at a buffer concentration of 25 mmol/L). During the storage at 60°C of the GHRP-6 formulations at 2.5 mg/mL, in sodium citrate buffer at 25 mmol/L, the purity values of the peptide were not affected by the concentration of sodium chloride present in the formulations, which was between 2.5 and 10 mg/mL. Hence, this compound may be used in the formulation as isotonizing agent. Table 1 summarizes the results of some of the formulations previously described.

**Table 1.** Purity percentage of GHRP-6 following storage at 60°C of aqueous formulations containing 2.5 mg of peptide per mL.

| Formulations | Time (days) | | | | |
|---|---|---|---|---|---|
| | 0 | 7 | 14 | 21 | 28 |
| Citrate-Phosphate at 100 mmol/L | 99.78 | 92.74 | 89.90 | 81.74 | 76.32 |
| Citrate at pH 100 mmol/L | 99.80 | 93.64 | 91.09 | 87.56 | 82.12 |
| Citrate at 25 mmol/L | 99.85 | 94.72 | 92.85 | 91.77 | 89.23 |
| Citrate at 25 mmol/L + 6 mg/mL of NaCl | 99.63 | 94.29 | 93.00 | 91.62 | 90.43 |

*The formulations indicated had a pH value of 6.0.*

Taking said results into consideration, the composition of the formulation to evaluate during the scaling up and the stability study was the following: GHRP-6 at 2.5 mg/mL, sodium citrate buffer at 25 mmol/L and pH between 5.7 and 6.3, while sodium chloride was used as isotonizing agent at 6 mg/mL.

*Preparation of a Liquid Formulation of GHRP-6 at 2.5 mg/mL in Citrate/Sodium Chloride: Scaling up and Stability Study*

Components of the GHRP-6 Formulation at 2.5 mg/mL: GHRP-6 (2.5 g), citric acid (4.8 g), sodium chloride (6.0 g) and water for injection (quantity sufficient for 1.0 L).

All the components of the formulation, with the exception of the API constituted by GHRP-6, were measured and dissolved with water for injection. The pH of the solution was checked and adjusted to the value of $6.0 \pm 0.3$ with NaOH at 1.0 mol/L. The API was added until reaching the desired concentration (2.5 mg/mL) in that solution. Next, the formulation was sterilized by filtration (through filters of 0.22 $\mu$m), in a class 100 area. Then, the sterile liquid formulation was dispensed in vials which were placed stoppers and seals, in a class 100 area. Samples from the three batches were placed in cold chamber, at controlled temperature ($5 \pm 3$°C), and in an incubator (for study of accelerated stability), at $25 \pm 2$°C. At given time intervals, samples were collected for analyzing the critical quality attributes: appearance of the product, GHRP-6 purity measured by RP-HPLC, GHRP-6 identity measured by ESI-MS, peptide's concentration determined by absorbance at 280 nm, pH, sterility and pyrogenicity. The analytical tests were made as previously described or as described in monograph USP-United States Pharmacopoeia.

During the storage of the GHRP-6 liquid formulation at 2.5 mg/mL in citrate/sodium chloride, during 12 months at $5 \pm 3$°C, the percentage of the fraction of the intact peptide remained above the 98.04%. During the stability study, the product remained without changes in its appearance. Also, the tests of sterility, pyrogens and identity by ESI-MS turned out satisfactory for all the batches, both at the beginning and at the end of the study. Similarly, the formulation remained stable during the storage at $25 \pm 2$°C, during six months. Purity measured by RP-HPLC remained above the 95.73% and tests of sterility, pyrogens and identity by ESI-MS turned out satisfactory for all the batches, at the beginning and at the end of the study. It is concluded that the formulation obtained turned out to be stable during storage for one year at $5 \pm 3$°C and during six months at $25 \pm 2$°C.

**Example 2. Studies of preclinical Toxicology conducted to the Liquid Formulation of GHRP-6 in Citrate/ Sodium Chloride**

In the preclinical studies described below, there was used the liquid formulation of GHRP-6 described in Example 1, which consists in GHRP-6 at 2.5 mg/mL, sodium citrate buffer at 25 mmol/L and pH between 5.7 and 6.3; sodium chloride at 6 mg/mL was used as isotonizing agent.

*Evaluation of Acute Toxicity in Sprague-Dawley Rats*

There was used a total of 50 rats (25 females and 25 males), which made up five groups of ten animals each. The dosage corresponding to each group was administered divided into three applications, one every six hours. The formulation was applied by intraperitoneal route, on days 1 and 2 of the experimental phase, according to the design shown in Table 2. The therapeutic dosage (TD) of 400 $\mu$g/kg was used as reference. The parameters evaluated were body weight, food consumption, clinical and macroscopic observations, as well as histopathological study of the organs of interest.

**Table 2.** Distribution of animals by groups and doses of the peptide used.

| Group | Treatment | GHRP-6 Dose |
|---|---|---|
| I | Saline Solution | - |
| II | Placebo | - |
| III | Formulation GHRP-6 | 20 times the TD (8000 $\mu$g/kg) |
| IV | Formulation GHRP-6 | 30 times the TD (12000 $\mu$g/kg) |
| V | Formulation GHRP-6 | 50 times the TD (20000 $\mu$g/kg) |

Following the detailed analysis of the results obtained, there was concluded that the formulation of GHRP-6 in citrate/sodium chloride, at high doses, did not cause acute systemic alterations that could compromise the safety of the patients. The evaluated formulation exhibited an adequate safety framework, up to 30 times the TD of the formulation of GHRP-6 in citrate/sodium chloride, for its use in clinical studies. Nevertheless, findings were reported in the adrenal glands (focal areas of necrosis at the level of the fascicular zone, with presence of tumescent cells and macro-vesicular steatosis), which are considered elements of toxicity -events that were related to the administration of high doses of GHRP-6 (in this case, 50 times the TD).

*Evaluation of Toxicity at Repeated Doses in Sprague-Dawley Rats*

There were used 100 young adult rats of both sexes (50 females and 50 males), which made up seven groups. The non-treated group and the Satellite Group (which was administered nine times the TD) were made completed with ten animals each. The other five groups were completed with 16 animals each. The design of the study appears summarized in Table 3. The Satellite Group was administered the highest dosage used in the study, with the purpose of evaluating the reversibility of the potential effects. The animals of this Group were sacrificed after twice the time with respect to the rest of the animals. There were conducted 14 consecutive administrations -one on a daily frequency, during two weeks-, by intraperitoneal route, in all the groups of treatment constituted. The parameters evaluated were body weight, food consumption, clinical and macroscopic observations, as well as histopathological study of the organs of interest. Likewise, there were made determinations of hematology and biochemistry, as well as of absolute and relative weights of the organs. Also, all the organs of each animal were studied in histopathology.

**Table 3.** Distribution of the treatments of the study by groups of animals.

| Group | Number of Animals | Treatment | GHRP-6 Dosage |
|---|---|---|---|
| I | 10 | Not treated | 0 |
| II | 16 | Saline Solution | 0 |
| III | 16 | Placebo | 0 |
| IV | 16 | Formulation GHRP-6 | 3 times the TD (1200 $\mu$g/kg) |
| V | 16 | Formulation GHRP-6 | 6 times the TD (2400 $\mu$g/kg) |
| VI | 16 | Formulation GHRP-6 | 9 times the TD (3600 $\mu$g/kg) |
| VII | 10 | Satellite, Formulation GHRP-6 | 9 times the TD (3600 $\mu$g/kg) |

From the study, there was concluded that the formulation of GHRP-6 in citrate/sodium chloride is not toxic at systemic level, at the doses evaluated.

*Evaluation of Local Tolerance in F1 Rabbits*

There was used a total of 18 female animals, which made up six groups of three animals each. There were executed five consecutive administrations by intravenous route, one every 72 hours. The distribution of the groups of animals per treatment was the following: Group I, not treated; Group II, placebo; Group III, TD of GHRP-6 (400 μg/kg); Group IV, five times the TD of GHRP-6 (2000 μg/kg); Group V, ten times the TD of GHRP-6 (4000 μg/kg); and Group VI (Satellite), ten times the TD of GHRP-6 (4000 μg/kg). The parameters evaluated were body weight, food consumption, clinical and macroscopic observations, as well as histopathological study of the organs of interest. Results proved that the repeated administration of the formulation of GHRP-6 in citrate/sodium chloride is well tolerated at high doses and that the adverse events found do not constitute signs of toxicity.

**Example 3. Dosage Study of the Liquid formulation of GHRP-6 in Citrate/Sodium Chloride, in Healthy Volunteers**

There was made a phase I, non-controlled clinical trial of dosage scaling up, in healthy volunteers, in which GHRP-6 was evaluated in the liquid formulation containing citrate/sodium chloride, developed in Example 1. The formulation contained: GHRP-6 at 2.5 mg/mL, sodium citrate buffer at 25 mmol/L and pH between 5.7 and 6.3; sodium chloride at 6 mg/mL was used as isotonizing agent. The peptide was administered at single dosage by intravenous route, at six dosage scales: 1, 10, 50, 100, 200 and 400 μg/kg of body weight. The sample was made up by 18 healthy subjects, of male sex, between 18 and 35 years of age, with a body weight within the normal limits, who voluntarily accepted to participate in the research. At each level of dosage scaling up, three subjects were treated. The transfer to the next level depended on the absence of toxicity (serious adverse event).

As to the peptide's safety, no serious adverse events were reported. There were recorded six different adverse events in 12 subjects (66.7%), sweating (50%), bradycardia (44.4%) and sleepiness (16.7%) being the most frequent ones, as observed in Table 4. All the adverse events exhibited a causal relation with the pharmaceutical product administered, which contained GHRP-6 as API. The number of adverse events reported increased with the dosage level of the peptide under study.

**Table 4.** Frequency of individuals with adverse events according to dosage group.

| Adverse Event (AE) | 1 μg/kg | 10 μg/kg | 50 μg/kg | 100 μg/kg | 200 μg/kg | 400 μg/kg |
|---|---|---|---|---|---|---|
| Bradycardia | 0 (0.0%) | 0 (0.0%) | 1 (33.3%) | 2 (66.7%) | 2 (66.7%) | 3 (100.0%) |
| Sweating | 0 (0.0%) | 2 (66.7%) | 1 (33.3%) | 1 (33.3%) | 2 (66.7%) | 3 (100.0%) |
| Tachycardia | 0 (0.0%) | 0 (0.0%) | 0 (0.0%) | 0 (0.0%) | 0 (0.0%) | 1 (33.3%) |
| Slight Fever | 1 (33.3%) | 0 (0.0%) | 0 (0.0%) | 0 (0.0%) | 0 (0.0%) | 0 (0.0%) |
| Sleepiness | 0 (0.0%) | 0 (0.0%) | 0 (0.0%) | 0 (0.0%) | 0 (0.0%) | 3 (100.0%) |
| **Total of Individuals with AE** | 1 (33.3%) | 2 (66.7%) | 2 (66.7%) | 2 (66.7%) | 2 (66.7%) | 3 (100.0%) |

There were observed values of cardiac frequency below the normal range (60 to 100), from the basal values. The pathological values (below 60) were reported as adverse events of bradycardia. The same had a duration that oscillated between 55 minutes and three hours, approximately, being the decrease of the values of cardiac frequency in Groups V (200 μg/kg) and VI (400 μg/kg) in the first 30 minutes elapsed from the administration of the peptide the ones of highest magnitude. With the adverse event that demanded most clinical attention (bradycardia), there was more specifically explored the relation with the concentration of GHRP-6. Even though no statistically significant correlation was detected, there was interesting to observe that, as the values of concentration of GHRP-6 increased, the greater the tendency towards bradycardia, given that cardiac frequencies were lower. Clinically, bradycardia may lead to serious problems when the peptide is administered in patients. In this study, it manifested in four out of the six dosage levels studied, despite the subjects being healthy volunteers.

Based on the results obtained in the dosage scaling up, in healthy individuals, there became necessary to explore new alternatives of formulation in which the peptide remained concentrated and stable, for its evaluation in humans.

**Example 4. Obtention of a New Formulation of GHRP-6**

*Effect of Tartrate, Succinate and Histidine Buffers at pH 5.0 - 6.0 on the Stability of GHRP-6 in Aqueous Solution*

Since the peptide is stable in the pH range between 5.0 and 6.0, there were studied the tartrate, succinate and histidine buffers, which exhibit a good buffer capacity for regulating pH in the value range. The buffers were prepared at a final concentration of 50mmol/L. For preparing the tartrate buffer, tartaric acid was used. Histidine buffer was prepared from the histidine-HCl reagent and the pH adjustment was made between 5.0 and 6.0 with NaOH at 1.0 mol/L, in both cases. Succinate buffer was prepared the same as the tartrate buffer, from succinic acid and NaOH.

The API (GHRP-6) was dissolved at the different buffer solutions. The shaking time was lower than one hour, at $25 \pm 2°C$. The concentration of GHRP-6 was of 2.5mg/mL, determined by absorbance at 280 nm. Next, the samples were sterilized by filtration (in filters of 0.22 $\mu$m), under aseptic conditions. For evaluating the stability of the peptide, the samples were placed in incubator, under stress conditions at $60 \pm 2°C$, during 28 days. Percentage of the intact peptide, concentration of the peptide and pH were evaluated according to the methods previously described.

The stability of GHRP-6 increased with the increase of pH, in the succinate buffer, in the range evaluated (5.0 - 6.0). The highest stability in succinate was reached at pH 6.0, where there was found 74.31% of purity of the peptide, after the formulation having being stored for 28 days at 60°C. The peptide's purity was lower than the one reached under the same conditions for the tartrate and histidine buffers.

Table 5 presents the results of the stability study of GHRP-6 for the tartrate and histidine buffers, and there may be seen that the purity value in RP-HPLC, in the sodium tartrate buffer, was higher than the one found for histidine-HCl, at pH values between 5.5 and 6.0.

**Table 5.** Percentage of the fraction of intact GHRP-6 in aqueous formulations, in tartrate and histidine buffers, at pH 5.0 - 6.0, stored at $60 \pm 2°C$.

| Time (days) | Sodium Tartrate | | | Histidine-HCl | | |
|---|---|---|---|---|---|---|
| | pH 5.0 | pH 5.5 | pH 6.0 | pH 5.0 | pH 5.5 | pH 6.0 |
| 0 | 99.43 | 99.46 | 99.49 | 99.25 | 99.16 | 99.11 |
| 7 | 97.05 | 97.24 | 97.43 | 96.72 | 95.83 | 94.93 |
| 14 | 94.32 | 94.86 | 95.39 | 93.82 | 93.06 | 92.19 |
| 21 | 92.99 | 93.50 | 94.01 | 92.43 | 91.70 | 90.97 |
| 28 | 92.31 | 92.95 | 93.58 | 92.04 | 90.25 | 88.46 |

*Effect of the Excipients on the Stability of Lyophilized GHRP-6 at pH 5.5, in Tartrate and Histidine Buffers*

It is known that the formulations in solid state exhibit higher stability than those in aqueous solution. For reaching the required stability of GHRP-6, there were evaluated different excipients for the lyophilization in the tartrate and histidine-HCl buffers. The buffers, at pH 5.5 and a concentration of 25 mmol/L, were prepared as previously described. The excipients used were: sucrose, lactose, trehalose, mannitol, glycine, alanine and dextran 40. The same were dissolved at a final concentration of the 3% (w/v), in the buffers evaluated. The peptide was dissolved up to a concentration of 2.5 mg/mL, as previously described. Then, the formulations were sterilized by filtration, under aseptic environmental conditions, and subjected to lyophilization. There was applied a conventional lyophilization cycle that adjusts to a group of excipients *(Santana et al., Biologicals, 2014, 42: 322-333)*.

For the stability study, the samples of the lyophilized formulations were placed in incubator, under stress conditions, at $50 \pm 2°C$. The highest stability of the lyophilized peptide was found in the presence of trehalose, followed by dextran 40, as reflected in Table 6. The results were similar when sodium tartrate buffer and histidine-HCl buffer at pH 5.5 were used. Surprisingly, lyoprotective excipients broadly used in the lyophilization of peptides and proteins, like sucrose, lactose and mannitol, did not turn out efficacious for GHRP-6. On the other hand, Figure 1 shows the RP-HPLC profiles of lyophilized GHRP-6 in the presence of four excipients. It can be observed that the highest stability of the peptide was reached when lyophilized in the presence of lyoprotective excipient trehalose.

**Table 6.** Percentage of the fraction of intact peptide in samples of lyophilized GHRP-6 in different excipients stored during six months at $50 \pm 2°C$.

| Buffers | Excipients | | | | | | |
|---|---|---|---|---|---|---|---|
| | Sucrose | Lactose | Trehalos e | Mannit ol | Glycin e | Alanine | Dextran |
| Tartrate | 49.1 | 48.5 | 98.7 | 30.0 | 88.6 | 93.1 | 96.6 |
| Histidine | 43.8 | 46.4 | 98.4 | 29.7 | 96.6 | 91.1 | 97.7 |

There was surprising the high chemical stability of GHRP-6 lyophilized in the presence of tartrate and histidine buffers in combination with trehalose, with respect to the rest of the formulations evaluated. Likewise, these formulations exhibited a low number of sub-visible particles per volume unit, when analyzed applying the light obscuration method, with results 100 times lower than those demanded for particles higher than or equal to 10 $\mu$m and 25 $\mu$m, by the US pharmacopoeia *(USP < 788 >, 2008)*. Hence, the composition of the lyophilized formulation evaluated during the scaling up and the stability study was: peptide GHRP-6 at 2.5 mg/mL, sodium tartrate buffer at 25 mmol/L, pH from 5.0 to 6.0, and trehalose at the 3% (w/v) as stabilizing or isotonizing agent.

**Example 5. Scaling up and Stability Study of Liquid Formulations of GHRP-6 Tartrate/Trehalose**

There were developed two liquid formulations of GHRP-6 that contained tartaric acid at 3.75 g/l, α-α dihydrate trehalose at 33.16 g/l, water for injection at a quantity sufficient for 1.0 L. In one of the formulations, the peptide was at the concentration of 2.5 mg/mL and in the other, at 10.0 mg/mL. To obtain the concentration of 2.5 mg/mL, there were dissolved 2.5 g of GHRP-6, while for the concentration of 10.0 mg/mL, 10.0 g of GHRP-6 were dissolved.

All the components of the formulation, with the exception of the API of GHRP-6, were measured and dissolved in water for injection. The pH of the solution was checked and adjusted at the value of 5.5 ± 0.3 with NaOH at 1.0 mol/L. GHRP-6 was progressively added until the desired concentration (2.5 or 10.0 mg/mL) was obtained. Following this, the formulation was sterilized by filtration; the sterile liquid formulation was dispensed in vials, in a class 100 area. Three batches of the liquid formulation of the peptide were manufactured in tartrate/trehalose, at each concentration of GHRP-6 (2.5 and 10.0 mg/mL). Samples from the batches were placed in cold chamber, at controlled temperature (5 ± 3°C), or in incubator, at 25 ± 2°C. At given time intervals, the samples were analyzed. The stability study at 25 ± 2°C was conducted for six months. The critical quality attributes and the analytical methods were evaluated similar to those previously described.

Table 7 includes the results of the stability study of the liquid formulation of GHRP-6. As observed, during the storage for two years at 5 ± 3°C, the percentage of the fraction of the intact peptide remained above the 97%, at the concentration of 2.5 mg/mL, and above the 98%, at 10.0 mg/mL.

**Table 7.** Percentage of intact peptide of liquid formulations of GHRP-6, in tartrate/trehalose, following storage at two temperatures.

| Time (months) | GHRP-6 at 2.5 mg/mL | | GHRP-6 at 10.0 mg/mL | |
|---|---|---|---|---|
| | 5 ± 3 °C | 25 ± 2 °C | 5 ± 3 °C | 25 ± 2 °C |
| 0 | 99.42 ± 0.04 | 99.42 ± 0.04 | 99.48 ± 0.06 | 99.48 ± 0.06 |
| 3 | 99.31 ± 0.03 | 96.28 ± 0.03 | 99.28 ± 0.05 | 97.94 ± 0.09 |
| 6 | 99.28 ± 0.07 | 95.47 ± 0.04 | 99.05 ± 0.08 | 97.30 ± 0.12 |
| 12 | 98.41 ± 0.04 | - | 98.53 ± 0.04 | - |
| 24 | 97.85 ± 0.09 | - | 98.01 ± 0.06 | - |

*There are shown mean value and standard deviation.*

During the six-month storage at 25 ± 2°C, the percentage of the fraction of intact GHRP-6 remained above the 95%, for the presentation of 2.5 mg/mL, and above the 97%, for that of 10.0 mg/mL. Also, pH remained within the required range. The tests of appearance, count of sub-visible particles, sterility, pyrogens and identity by ESI-MS turned out satisfactory for all the batches, at the beginning and at the end of the study, for the two concentrations of the peptide and at the two temperature values studied. Based on the stability study, there was concluded that the liquid formulation evaluated was stable at 5 ± 3°C during 24 months and at 25 ± 2°C during six months.

**Example 6. Scaling up and stability study of lyophilized formulations of GHRP-6 in tartrate/trehalose**

It is known that proteins and peptides in solid stage generally exhibit higher stability than in liquid state, as storage temperature increases. Hence, there were developed two lyophilized formulations of GHRP-6 containing tartaric acid at 3.75 g/l, α-α-dihydrate trehalose at 33.16 g/l, water for injection at quantify sufficient for 1.0 L. For the concentration of 2.5 mg/mL, there were dissolved 2.5 g of GHRP-6, and for the concentration of 10.0 mg/mL, 10.0 g of GHRP-6 were dissolved. There was applied the same manufacturing method described in Example 5. Three batches of the in-bulk product in liquid state were prepared. Next, the in-bulk product was dispensed in vials, for proceeding to the lyophilization process. There was applied a conventional lyophilization cycle *(Santana et al., Biologicals, 2014, 42: 322-333).* Based on the procedure described, there were manufactured three batches of the lyophilized formulation of GHRP-6 in tartrate/trehalose at each strength of GHRP-6 (2.5 or 10.0 mg/vial). Samples collected from the batches were placed in cold chamber, at controlled temperature (5 ± 3°C), or in incubator, at 25 ± 2°C. At given time intervals, the samples of the lyophilized formulation were analyzed. The stability study at 25 ± 2°C was conducted during six months. The critical quality attributes and the analytical methods evaluated were similar to those previously described.

Table 8 includes the results of the stability of the lyophilized formulations of GHRP-6. During the two-year storage at 5 ± 3°C, the percentage of the fraction of the intact peptide remained above the 99%, for both presentations (2.5 mg/vial and 10.0 mg/vial). Concentration of the peptide, residual moisture and pH of all the samples corresponding to both formulations remained at adequate values during the time interval studied.

**Table 8.** Percentage of intact peptide in the lyophilized formulations of GHRP-6 in tartrate/trehalose, following the storage at two temperatures.

| Time (months) | GHRP-6 at 2.5 mg/vial | | GHRP-6 at 10.0 mg/vial | |
|---|---|---|---|---|
| | 5 ± 3 °C | 25 ± 2 °C | 5 ± 3 °C | 25 ± 2 °C |
| 0 | 99.51 ± 0.04 | 99.51 ± 0.04 | 99.61 ± 0.16 | 99.61 ± 0.16 |
| 3 | 99.43 ± 0.06 | 99.44 ± 0.28 | 99.52 ± 0.05 | 99.45 ± 0.05 |
| 6 | 99.57 ± 0.19 | 99.40 ± 0.02 | 99.50 ± 0.08 | 99.42 ± 0.11 |
| 12 | 99.33 ± 0.09 | - | 99.59 ± 0.04 | - |
| 24 | 99.24 ± 0.07 | - | 99.36 ± 0.09 | - |

*Mean value and standard deviation are shown.*

During the six-month storage at 25 ± 2°C, the percentage of the fraction of intact GHRP-6 remained above the 99.4%, for both presentations. Concentration of the peptide, residual moisture and pH of all the samples remained at adequate values during the time interval studied. Also, the tests of appearance, count of sub-visible particles, sterility, pyrogens and identity by ESI-MS turned out satisfactory for all the batches, at the beginning and at the end of the study, for the two concentrations of the peptide and at the two temperature values studied.

Based on this stability study, there was concluded that the lyophilized formulation evaluated was very stable at 5 ± 3°C for 24 months and at 25 ± 2°C for six months. The percentage of intact peptide remained above the 99% for both presentations, at 25 ± 2°C, during six months, which justifies that the peptide may be even stored without cold chain, during the distribution of the product.

**Example 7. Evaluation of the Biological Activity of the Formulation of GHRP-6 in Tartrate/Trehalose, in an Animal Model**

The *in-vivo* biological activity of two formulations of GHRP-6 that contained the peptide at a concentration of 2.5 mg/mL was evaluated. To that end, there were used samples of the formulation of GHRP-6 in citrate/NaCl (developed in Example 1) and of the formulation of GHRP-6 in tartrate/trehalose, prepared as described in Example 5, in which the peptide was at 2.5 mg/mL. As control, there were also evaluated the placebos corresponding to each formulation, prepared in a similar manner, but without adding the GHRP-6 peptide.

The inotropic activity of the peptide was evaluated in Balb/c male mice (6 animals per group). There was administered a dosage of 100 μg/kg of the GHRP-6 formulations or the equivalent volume of the placebos, by intraperitoneal route. For each group, echocardiograms were obtained on time zero and every five minutes after the administration, until the 15 minutes, under the same conditions of monitoring, temperature and sedation.

The two-dimensional ultrasonic images in M mode were obtained using a high-resolution image system (Vevo 770™, Visual Sonics Inc.). The system was equipped with a scaled-up matrix linear transductor of 40 MHz, with data acquisition at frequencies of up to 250 photograms per second, with an axial resolution of up to 30 microns among focal zones. Data processing and analysis were made with the application of software Vevo 770 (version 3.0.0, Visual Sonics Inc.), in a view of the long parasternal axis, in a 45° angle, and the caudal angulation of the platform. The left ventricular ejection fraction (LVEF) was calculated by means of the Teichold equation:

$$LVEF = \frac{FDV - FSV}{FDV} * 100$$

Where:

FDV: Final diastolic volume
FSV: Final systolic volume.

The measurements were taken in accordance with the specific echocardiogram: the diastolic parameters were measured in the peak of R wave and the systolic parameters, in the peak of T wave. There were completed from three to five consecutive cardiac cycles, following the recommendations of the American Society of Echocardiography (Langy et al., EUR. J. Echocardiogr., 2006, 7 (2): 79-108).

According to Figure 2, for the animals treated with the peptide in the formulation in tartrate/trehalose, LVEF significantly increased over time, and remained increased for at least the 15 minutes studied. This effect was not observed in the animals treated with the placebo of the said formulation. LVEF was higher in the case of the animals that received the formulation in tartrate/trehalose, as compared to the animals treated with the other formulation of the peptide. In summary, the formulation of GHRP-6 in tartrate/trehalose exhibited higher inotropic activity in the animal model used.

**Example 8. Studies of Preclinical Toxicology of a Formulation of GHRP-6 in Tartrate/Trehalose**

Once the desired biological activity had been proved, the lyophilized formulation containing sodium tartrate, trehalose and 5 mg of GHRP-6 per vial was reconstituted and subjected to toxicology studies, in the following animal models.

*Evaluation of Acute Toxicity in Sprague-Dawley Rats*

There was used a total of 50 rats (25 females and 25 males) which completed five groups of ten animals each. The dosage corresponding to each group was administered divided into three applications, one every six hours. The product was applied by intraperitoneal route, on days 1 and 2 of the experimental phase. The TD of 400 $\mu$g/kg was taken as reference. Table 9 shows the design of the study conducted.

**Table 9.** Distribution per groups of animals and doses of the peptide used.

| Group | Treatment | GHRP-6 Dosage |
|---|---|---|
| I | Saline Solution | - |
| II | Placebo | - |
| III | Formulation GHRP-6 | 30 times the TD (12000 $\mu$g/kg) |
| IV | Formulation GHRP-6 | 55 times the TD (22000 $\mu$g/kg) |
| V | Formulation GHRP-6 | 100 times the TD (40000 $\mu$g/kg) |

After the detailed analysis of the results obtained, there was concluded that the lyophilized formulation of peptide GHRP-6 in tartrate/trehalose, at the doses evaluated, caused no acute systemic alterations that compromised the safety of the patients. The same exhibited an adequate safety framework of up to 100 times the TD, hence, the formulation in tartrate/trehalose could be subjected to clinical studies.

*Evaluation of Toxicity at Repeated Doses in Sprague-Dawley Rats*

There were used young adult 100 rats of both sexes (50 females and 50 males), which made up seven groups. Table 10 shows the design of the study conducted. The non-treated group and the Satellite Group (which was administered 50 times the TD) were made up by ten animals each. The other five groups were completed with 16 animals each. The Satellite Group was administered the highest dosage used in the study, with the purpose of evaluating the reversibility of the potential effects, and the animals of the said Group were sacrificed after twice the time with respect to the rest of the animals of the study. There were made 14 consecutive administrations, one on a daily basis during two weeks, by intraperitoneal route, in all the groups of treatment constituted.

**Table 10.** Distribution of animals per treatment under study.

| Group | Number of Animals | Treatment | GHRP-6 Dosage |
|---|---|---|---|
| I | 10 | Not treated | 0 |
| II | 16 | Saline Solution | 0 |
| III | 16 | Placebo | 0 |
| IV | 16 | Formulation GHRP-6 | 10 times the TD (4000 $\mu$g/kg) |
| V | 16 | Formulation GHRP-6 | 30 times the TD (12000 $\mu$g/kg) |
| VI | 16 | Formulation GHRP-6 | 50 times the TD (20000 $\mu$g/kg) |
| VII | 10 | Satellite, Formulation GHRP-6 | 50 times the TD (20000 $\mu$g/kg) |

After the detailed analysis of the results obtained, there was concluded that the lyophilized formulation of GHRP-6 in tartrate/trehalose was not toxic at systemic level, until 50 times the TD.

*Evaluation of Local Tolerance in F1 Rabbits*

There was used a total of 18 female animals, which made up six groups of three animals each. There were made five consecutive administrations, by intravenous route, one every 72 hours. The distribution of the groups of animals per treatment was the following: Group 1, not treated; Group II, placebo; Group III, TD of GHRP-6 (400 $\mu$g/kg); Group IV, five times the TD of GHRP-6 (2000 $\mu$g/kg); Group V, 10 times the TD of GHRP-6 (4000 $\mu$g/kg); and Group VI, 10 times the TD of GHRP-6 (4000 $\mu$g/kg).

Once there was completed the analysis of the results obtained in this study, there was concluded that the repeated administration of the formulation of GHRP-6 in tartrate/trehalose was well tolerated and that the adverse events found did not constitute signs of toxicity.

**Example 9. Evaluation of the Safety of the Formulation of GHRP-6 in Tartrate/Trehalose in Patients with Acute Myocardial Infarction**

In this study, there was used the lyophilized formulation of GHRP-6 that contained sodium tartrate, trehalose and 5 mg of GHRP-6 per vial. There was conducted a phase I-B clinical trial (exploratory), prospective, multicenter, open and randomized, in patients with acute myocardial infarction, who were subjected to primary percutaneous transluminal coronary angioplasty. Once the lyophilized formulation was reconstituted, the peptide was administered at random, at the doses of 50 or 100 $\mu$g/kg of body weight of the patient, by intravenous route, in bolus. The first dosage was administered at the moment of the patient's inclusion in the study. Afterwards, the product was administered every 12 hours, during seven days, until a total of 14 administrations of the peptide. The sample was constituted by 19 patients of both sexes, older than 30 years, who participated voluntarily in the research. The primary objective of the study was to evaluate the safety of the lyophilized formulation containing GHRP-6.

Table 11 presents a summary of the adverse events registered, with the intention of comparing against the ones found in the study of dosage scaling up in healthy volunteers, conducted with the formulation of GHRP-6 in citrate/NaCl. In the said Table, it is seen that in this study, there were not observed the adverse events caused by the formulation of citrate/NaCl reflected in Example 3. The rest of the events reported were of mild or moderate intensities; most of the same exhibited low causality relation with the peptide object of study, and were attributed to the baseline disease of the patients.

**Table 11.** Frequency of individuals with adverse events according to dosage group.

| Adverse Events (AEs) | Group I (50 $\mu$g/kg) | Group II (100 $\mu$g/kg) | Total (Group I + II) |
|---|---|---|---|
| N | 9 | 10 | 19 |
| Sweating | 3 (33.3%) | 1 (10.0%) | 4 (21.0%) |
| Bradycardia | 0 (0.0%) | 0 (0.0%) | 0 (0.0%) |
| Tachycardia | 0 (0.0%) | 0 (0.0%) | 0 (0.0%) |
| Slight Fever | 0 (0.0%) | 0 (0.0%) | 0 (0.0%) |
| Sleepiness | 0 (0.0%) | 0 (0.0%) | 0 (0.0%) |

Therefore, the clinical evaluation of the formulation of GHRP-6 in tartrate/trehalose concluded that the same does not produce bradycardia, which allows its use in the treatment of patients suffering from cardiovascular or cerebrovascular diseases, and that it exhibits the required stability for at least two years, at a high concentration of the peptide.

**Claims**

1. A pharmaceutical composition comprising growth-hormone releasing peptide 6 (GHRP-6) at a concentration between 1.0 mg/mL and 10.0 mg/mL, tartrate buffer at pH 5.0 - 6.0 and trehalose.

2. The composition according to claim 1 wherein the buffer has a concentration between 10 mmol/L and 100 mmol/L.

3. The composition according to claim 1 wherein the tartrate buffer is formed by a sodium salt, potassium salt or a mixture of both.

4. The composition according to claim 1 wherein trehalose is in the range between 2% and 10% (w/v).

5. The composition according to claim 1 which is in liquid form or is the result of suspending a lyophilisate.

6. The pharmaceutical composition of any of claims from 1 to 5 for use as a medicament.

7. The pharmaceutical composition of any of claims from 1 to 5 for use as cytoprotective, cardio-protective, cardio-restorer or neuroprotective agent, or as restorer of cerebral damage.

8. The pharmaceutical composition of any of claims from 1 to 5 for use as a medicament, wherein the pharmaceutical composition is administered by intravenous, subcutaneous or intramuscular routes.

9. A kit of parts comprising the pharmaceutical composition of any of claims from 1 to 5.

10. The kit of parts according to claim 9, wherein said kit of parts additionally comprises a second pharmaceutical composition.

**Patentansprüche**

1. Pharmazeutische Zusammensetzung, umfassend Wachstumshormon-freisetzendes Peptid 6 (GHRP-6) in einer Konzentration zwischen 1,0 mg/ml und 10,0 mg/ml, Tartratpuffer bei pH 5,0-6,0 und Trehalose.

2. Zusammensetzung nach Anspruch 1, wobei der Puffer eine Konzentration zwischen 10 mmol/L and 100 mmol/L hat.

3. Zusammensetzung nach Anspruch 1, wobei der Tartratpuffer aus einem Natriumsalz, Kaliumsalz oder einer Mischung aus beiden gebildet ist.

4. Zusammensetzung nach Anspruch 1, wobei Trehalose im Bereich zwischen 2 % und 10 % (w/v) ist.

5. Zusammensetzung nach Anspruch 1, die in flüssiger Form ist oder das Ergebnis des Suspendierens eines Lyophilisats ist.

6. Pharmazeutische Zusammensetzung nach einem der Ansprüche 1 bis 5 zur Verwendung als Arzneimittel.

7. Pharmazeutische Zusammensetzung nach einem der Ansprüche 1 bis 5 zur Verwendung als zytoprotektives, kardioprotektives, kardiorestaurierendes oder neuroprotektives Mittel oder zur Behebung von Hirnschäden.

8. Pharmazeutische Zusammensetzung nach einem der Ansprüche 1 bis 5 zur Verwendung als Arzneimittel, wobei die pharmazeutische Zusammensetzung auf intravenösem, subkutanem oder intramuskulärem Weg verabreicht wird.

9. Teilesatz, umfassend die pharmazeutische Zusammensetzung nach einem der Ansprüche 1 bis 5.

10. Teilesatz nach Patentanspruch 9, wobei der Teilesatz zusätzlich eine zweite pharmazeutische Zusammensetzung umfasst.

**Revendications**

1. Composition pharmaceutique comprenant le peptide 6 libérant l'hormone de croissance (GHRP-6) à une concentration comprise entre 1,0 mg/ml et 10 mg/ml, du tampon tartrate à pH 5,0 - 6,0 et du tréhalose.

2. Composition selon la revendication 1, dans laquelle le tampon a une concentration comprise entre 10 mmoles/l et 100 mmoles/l.

3. Composition selon la revendication 1, dans laquelle le tampon tartrate est formé par un sel de sodium, un sel de potassium ou un mélange des deux.

4. Composition selon la revendication 1, dans laquelle le tréhalose est dans la plage comprise entre 2 % et 10 % (p/v).

**5.** Composition selon la revendication 1, qui est sous forme liquide ou est le résultat de la mise en suspension d'un lyophilisat.

**6.** Composition pharmaceutique selon l'une quelconque des revendications 1 à 5, pour utilisation en tant que médicament.

**7.** Composition pharmaceutique selon l'une quelconque des revendications 1 à 5, pour utilisation en tant qu'agent cytoprotecteur, cardioprotecteur, cardio-régénérateur ou neuroprotecteur, ou en tant que régénérateur de lésion cérébrale.

**8.** Composition pharmaceutique selon l'une quelconque des revendications 1 à 5, pour utilisation en tant que médicament, la composition pharmaceutique étant administrée par voie intraveineuse, sous-cutanée ou intramusculaire.

**9.** Ensemble de parties comprenant la composition pharmaceutique selon l'une quelconque des revendications 1 à 5.

**10.** Ensemble de parties selon la revendication 9, ledit ensemble de parties comprenant en outre une seconde composition pharmaceutique.

**Figure 1**

**A**

**Sodium Tartrate**

After 6 months at 50 ± 2 °C

GHRP-6

Glycine

Alanine

Dextran 40

Trehalose

Relative retention time

**B**

**Histidine-HCl**

After 6 months at 50 ± 2 °C

GHRP-6

Glycine

Alanine

Dextran 40

Trehalose

Relative retention time

**Figure 2**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **CASANUEVA** ; **DIEGUEZ**. *Trends Endocrinol. Metab.*, 1999, vol. 10, 30-38
- **KOJIMA et al.** *Nature*, 1999, vol. 402, 656-660
- **BODART et al.** *Circ. Res.*, 1999, vol. 85, 796-802
- **MURRIEL** ; **MOCHLY**. *Arch. Biochem. Brophys.*, 2003, vol. 420, 246-254
- **COLONNA et al.** *EUR. J. Pharmacol.*, 1997, vol. 334, 201-207
- **CIBRIÁN et al.** *Clin. Sci.*, 2006, vol. 110, 563-573
- **BERLANGA et al.** *Clin. Sci.*, 2007, vol. 112, 241-250
- **BERLANGA et al.** *Clin. Med. Insights Cardiol.*, 2017, vol. 11, 1-9
- **SUBIRÓS et al.** *Neurol. Res.*, 2016, vol. 38 (3), 187-195
- **LEAL-CERRO et al.** *EUR. J. Endocrinol.*, 1995, vol. 132 (6), 712-715
- **LOCHE et al.** *J. Clin. Endocrinol. Metab.*, 1995, vol. 80 (2), 674-678
- **AJAYI et al.** *J. Clin. Pharmacol.*, 2000, vol. 40 (10), 1093-1101
- **CHOONARA et al.** *Paediatr. Perinat. Drug Ther.*, 2001, vol. 5, 12-18
- **HA et al.** *Int. J. Pharm.*, 1996, vol. 144, 91-97
- Solid phase peptide synthesis: a practical approach. **ATHERTON** ; **SHEPPARD**. Current Protocols in Protein Science. IRL Press, 1989
- **SANTANA et al.** *Biologicals*, 2014, vol. 42, 322-333
- **LANGY et al.** *EUR. J. Echocardiogr.*, 2006, vol. 7 (2), 79-108